# EUROPÄISCHE PATENTSCHRIFT

(11) **EP 2 990 013 B1**
(45) Veröffentlichungstag und Bekanntmachung des Hinweises auf die Patenterteilung: **21.06.2017**
(21) Anmeldenummer: 14183090.1
(22) Anmeldetag: 01.09.2014
(51) Int. Cl.: A61F 2/34, A61F 2/30

(54) **Künstliche Gelenkschale**
Artificial articulated shell
Cupule acétabulaire artificielle

(43) Veröffentlichungstag der Anmeldung: 02.03.2016
(73) Patentinhaber: Jossi Holding AG, 8546 Islikon (CH)
(72) Erfinder: Jossi, Armin, 8500 Frauenfeld (CH); Meyenhofer, Andreas, 8255 Schlattingen (CH); Schmidt, Martin, 8532 Warth (CH)
(74) Vertreter: Hepp Wenger Ryffel AG

(56) Entgegenhaltungen:
- EP-A1- 2 502 604
- WO-A2-2012/113690
- DE-A1- 3 101 333
- DE-A1- 3 130 732
- DE-A1- 19 755 536
- US-A1- 2011 251 698
- US-A1- 2013 190 889

## Beschreibung

Die vorliegende Erfindung betrifft eine künstliche Gelenkschale gemäss dem Oberbegriff von Anspruch 1. Die Merkmale des Oberbegriffs des Anspruchs 1 sind aus US 2011/0251698 A1 bekannt.

Beim Auftreten von Schädigungen an einer Gelenkpfanne, zum Beispiel am Acetabulum, eines Patienten ist es gängige Praxis, eine künstliche Gelenkpfanne zu implantieren. Solche Gelenkpfannen sind in verschiedenen Ausführungen bekannt. Einerseits können sie mehrteilig, beispielsweise aus einer äusseren Gelenkschale (Aussenschale) und einer inneren Gelenkschale (Innenschale), aufgebaut sein. Die Aussenschale dient dabei dem Einbetten der Gelenkpfanne in einen Knochen, während die Innenschale, welche in die Aussenschale eingesetzt wird, eine Lagerfläche für einen entsprechenden Gelenkkopf bildet. Andererseits sind auch einstückige künstliche Gelenkpfannen mit nur einer Schale bekannt. Gelenkpfannen beider Arten werden seit geraumer Zeit im klinischen Umfeld verwendet.

Erfolgt die Implantation einer Gelenkpfanne als vormontierte Einheit, beispielsweise von einer Aussenschale und einer Innenschale oder mit nur einer Schale, wird diese als sogenannte Monoblock-Pfanne bezeichnet. Derartige Gelenkpfannen vermindern das Risiko einer sich aus der Aussenschale lösenden Innenschale. Sie sind sehr stabil, aufgrund ihrer Bauweise und der erforderlichen Dicke der jeweiligen Materialien jedoch vergleichsweise voluminös.

Dies hat zur Folge, dass selbst bei relativ kleinen Beschädigungen der natürlichen Gelenkpfanne, zum Beispiel des Acetabulums, eines Patienten, speziell bei Beschädigungen des Knorpels bei intaktem Knochen, bei der Vorbereitung des Knochens für die Implantation relativ viel Material entfernt werden muss. Insbesondere bei jungen Patienten bedeutet dies, dass bei der Vorbereitung des Knochens für die Aufnahme der künstlichen Gelenkpfanne schon zu einem sehr frühen Zeitpunkt relativ viel Knochensubstanz verloren geht, so dass für eine zu einem späteren Zeitpunkt eventuell erforderlichen Reoperation nicht mehr so viel Knochensubstanz zur Verfügung steht.

Ein weiteres Problem bei herkömmlichen Gelenkschalen besteht darin, dass aufgrund der erforderlichen Wandstärke der Gelenkschale die Gelenkkugel im Vergleich zum natürlichen Gelenk verhältnismässig klein ausfällt. Dadurch wird die Charakteristik des Kugelgelenks nachteilig verändert. Ausserdem treten aufgrund der kleinen Gelenkkugel punktuell sehr hohe Belastungen im Gelenk auf. Zuletzt stellt die Dickwandigkeit von künstlichen Gelenkschalen insbesondere bei Patienten, bei welchen die Implantation einer sehr kleinen Gelenkschale erforderlich ist, ein Problem dar.

Durch die EP 1 025 815 A1 ist eine künstliche Gelenkschale zur Implantation in eine entsprechende Kavität im Knochen eines Patienten bekannt, die eine Wandstärke von ca. 2,5 mm bis 3 mm aufweist. Die besagte Gelenkschale ist aus Metall, insbesondere aus einer Kobalt-Chrom-Legierung, hergestellt. Des Weiteren können sowohl die Innenfläche als auch die Aussenfläche der besagten Gelenkschale mit verschiedenen Materialien beschichtet sein.

Allerdings ist bei einer derartigen Gelenkschale aufgrund der geringen Wandstärke die erforderliche mechanische Stabilität der Schalenstruktur in Metallbauweise nur schwierig zu erreichen. Des Weiteren stellt das Gleitverhalten des Gelenkkopfes an der Innenseite der metallischen Schale je nach Wahl des Materials des Gelenkkopfes ein Problem dar, wodurch die Innenseite der Gelenkschale unter Umständen mit einem weiteren nichtmetallischen Material beschichtet werden muss. Das Auftragen einer besonders glatten Beschichtung an der Innenseite der Gelenkschale ist allerdings nur mit erheblichem Aufwand zu erzielen. Ein weiteres Problem stellt das stabile Einbetten der Schale in einen Knochen eines Patienten dar. Ein stabiler Halt ist nicht immer zu gewährleisten. Ferner ist die biologische Verträglichkeit des Schalenmaterials, insbesondere auf der Aussenseite, teilweise nicht gegeben.

Es ist daher die Aufgabe der Erfindung, die Nachteile des Standes der Technik zu überwinden.

Insbesondere ist es die Aufgabe der Erfindung, eine Gelenkschale in Monoblockbauweise zu schaffen, die in einem Hüftknochen mit möglichst geringem Verlust an Knochenmaterial eingesetzt werden kann. Dennoch soll eine erfindungsgemässe Gelenkschale die Verwendung eines Gelenkkopfes, der im Wesentlichen denselben Durchmesser wie ein natürlicher Gelenkkopf aufweist, ermöglichen. Die Gelenkschale soll auch in sehr kleinem Durchmesser realisierbar und damit zur Implantation in besonders kleinen Patienten geeignet sein. Dennoch soll die Gelenkschale eine sehr hohe mechanische Stabilität gewährleisten. Des Weiteren soll eine erfindungsgemässe Gelenkschale für einen Patienten gut verträglich sein, insbesondere eine hohe Biokompatibilität aufweisen. Vor allem aber soll sie sicher in einer Knochenstruktur verankerbar sein und innerhalb von kurzer Zeit eine feste Verbindung mit dem Knochen eines Patienten eingehen.

Diese Aufgaben werden durch eine Gelenkschale gelöst, welche die Merkmale im Anspruch 1 aufweist.

Die Gelenkschale hat insbesondere im Wesentlichen die Form einer Kugelkalottenschale, mit einer konvexen Aussenfläche und einer konkaven Innenfläche. Ausserdem weist die Gelenkschale einen äusseren Durchmesser (OD) und einen inneren Durchmesser (ID) auf. Der äussere Durchmesser (OD) bezieht sich in diesem Zusammenhang auf den Durchmesser einer Kugel, deren Oberfläche dieselbe Krümmung wie die mittlere Krümmung der konvexen Aussenfläche einer erfindungsgemässen Gelenkschale hat. Der innere Durchmesser (ID) bezieht sich auf den Durchmesser einer Kugel deren Oberfläche der konkaven Innenfläche der vorliegenden Gelenkschale entspricht. Das Verhältnis der Differenz (D) zwischen dem äusseren Durchmesser (OD) und dem inneren Durchmesser (ID) zum äusseren Durchmesser (OD) liegt dabei in einem Bereich zwischen 0.2 und 0.1.

Bei einem äusseren Schalendurchmesser (OD) von 4.0 cm würde dies bei einer entsprechenden Kugelkalottenschale einer Wandstärke in einem Bereich zwischen 4 mm und 2 mm entsprechen. Bei einem äusseren Schalendurchmesser (OD) von 9.0 cm würde die Wandstärke entsprechend in einem Bereich zwischen 9 mm und 4.5 mm liegen.

Die Erfindung zeichnet sich dadurch aus, dass die Gelenkschale aus einem Keramikmaterial gefertigt ist und die konvexe Aussenfläche eine Makrostrukturierung aufweist.

Durch die Verwendung von Keramik als Schalenmaterial ist es möglich, eine sehr dünnwandige Gelenkschale mit besonders hoher mechanischer Festigkeit herzustellen. Der Verlust am Knochenmaterial bei der Implantation einer derartigen Schale bei einem Patienten ist daher minimal. Ausserdem ermöglicht es eine solche Schale ein künstliches Kugelgelenk bereitzustellen, bei dem der Gelenkkopf im Wesentlichen dieselbe Grösse aufweist wie bei einem entsprechenden natürlichen Gelenk. Eine möglichst natürliche Gelenkcharakteristik wird damit erzielt. Ausserdem wird das Auftreten von sehr hohen mechanischen Belastungen, wie sie bei kleineren Gelenkkugeln sehr oft vorkommen, vermieden.

Des Weiteren weist eine derartige Schale eine besonders hohe Körperverträglichkeit, insbesondere eine hervorragende Biokompatibilität auf. Durch die Wahl von Keramik als Schalenmaterial kann die Gelenkpfanne problemlos in einem Knochen eines Patienten implantiert werden, wobei es innerhalb kurzer Zeit zu einer festen Verbindung der Aussenfläche der Schale mit der Knochenstruktur kommt.

Eine zentrale Rolle spielt dabei die Makrostrukturierung auf der konvexen Aussenfläche. Durch sie wird die äussere Oberfläche der Gelenkschale signifikant erhöht. Dies begünstigt zusätzlich das Eingehen einer festen Verbindung der Gelenkschale mit dem Knochen, in welchen sie implantiert wird.

Die konkave Innenfläche kann eine Lagerfläche für eine in der Gelenkschale aufzunehmende Gelenkkugel bilden. Durch diesen Monoblockaufbau benötigt die Schale weder eine Innenschale (Inlay), noch muss die konkave Lagerfläche im Zuge des Herstellungsverfahrens beschichtet werden. Gängige Materialkombinationen für die Lagerfläche und die Gelenkkugel können verwendet werden, wodurch ein optimales Gleitverhalten des Gelenkes gewährleistet ist. Alternativ kann die konkave Innenfläche allerdings auch eine Haltefläche für eine in der Gelenkschale aufzunehmende Innenschäle bilden. Eine entsprechend aufgebaute Gelenkpfanne kann nur als Aussenschale oder als Monoblock implantiert werden. Durch diese vielseitige Anwendbarkeit lässt sich eine erfindungsgemässe Gelenkschale in einer grossen Breite von medizinischen Indikationen verwenden.

Die Makrostrukturierung auf der konvexen Aussenfläche der Gelenkschale kann eine Vielzahl von Strukturelementen umfassen. Diese können durch Vertiefungen, vorzugsweise durch Materialabtragungen, begrenzt sein und eine Grösse von 0.3 mm bis zu 3 mm haben. Je nach Oberflächenstrukturierung ist eine sehr effiziente Fertigung einer derartigen Gelenkschale möglich, da die Oberfläche beispielsweise nach Herstellung eines Grünlings spanabhebend, schleifend, ätzend, durch Sandstahlen, mittels Laserablation oder mit anderen materialabtragenden Methoden bearbeitet werden kann. Eine weitere Möglichkeit besteht ferner darin, dass eine Makrostrukturierung der Oberfläche bereits beim Formen der keramischen Gelenkschale gebildet wird. Als mögliches Herstellungsverfahren kommt hierzu beispielsweise Schlickerguss in Frage. Ebenfalls ist das Anbringen von Strukturelementen an einer glatteren Oberfläche möglich.

Allerdings kann die besagte Makrostrukturierung auch eine Porenstruktur mit einer Vielzahl von Hohlräumen umfassen, die eine Grösse von 0.03 mm bis zu 3 mm haben. Eine derartige poröse Oberflächenstruktur kann zum Beispiel durch Fertigung der Gelenkschale aus einem porösen Keramikmaterial oder durch Auftragen einer porösen äusseren Keramikschicht auf einem Rohling erzielt werden.

Eine derartige Makrostrukturierung kann zum Einwachsen der Trabekelstruktur eines Knochens geeignet sein. Bei der Trabekelstruktur, auch Substantia Spongiosa oder Spongiosa genannt, handelt es sich um ein schwammartig aufgebautes System aus feinen Knochenbälkchen, den sogenannten Trabekel, im Inneren eines Knochens. Durch Einwachsen dieser Knochenstruktur in die Makrostrukturierung der Gelenkschalenoberfläche wird eine sehr feste Verbindung (Osteointegration) zwischen Gelenkschale und Knochen ermöglicht.

In einer bevorzugten Ausführungsform einer erfindungsgemässen Gelenkschale können Vertiefungen, welche Strukturelemente in einer Reihe voneinander trennen, gegenüber Vertiefungen, welche Strukturelemente in einer benachbarten Reihe voneinander trennen, relativ zueinander versetzt angeordnet sein. Durch diese Anordnung lassen sich Oberflächenstrukturen schaffen, welche das Einbringen des Implantates in den Knochen vereinfachen. Des Weiteren ergibt sich eine Oberflächenstruktur, welche eine gute Verankerung des Implantates im Knochen ermöglicht. Zudem werden auf das Implantat wirkende Kräfte optimal auf das umliegende Knochengewebe verteilt.

Die Vertiefungen, insbesondere die Materialabtragungen, welche die Strukturelemente begrenzen, können auch durch sich überkreuzende Rillen mit entgegengesetzter Steigung gebildet sein. Auch durch diese Oberflächenstrukturierung wird eine gute Verankerung der Gelenkpfanne im Knochen ermöglicht. Des Weiteren ist eine derartige Strukturierung auf der Oberfläche einer Gelenkpfanne sehr einfach und kostengünstig anzubringen.

Die besagten Strukturelemente können als Quader, Pyramiden, Tetraeder, Keile, Polyeder oder in Form eines spitzwinkligen Satteldaches geformt sein. Die Form der Strukturelemente hängt von der Form der Vertiefungen, insbesondere der Materialabtragung ab. Durch entsprechende Wahl der Formgebung lassen sich so eine Vielzahl an Formen von Strukturelementen schaffen. Damit kann die Oberflächenstrukturierung der jeweiligen Art der Knochenstruktur angepasst werden, wodurch, abhängig von der vorliegenden medizinischen Indikation, eine ideale Verankerung des Implantates im Knochen erreicht werden kann.

Durch die Vertiefungen, insbesondere durch die Materialabtragungen, können konkave Oberflächenbereiche gebildet werden, deren Krümmung einen Krümmungskreisradius von mindestens 0.1 mm hat. Dies ist insbesondere bei der Fertigung der keramischen Gelenkschale von Bedeutung, wenn die Oberfläche eines Grünlings vor dem Sinterprozess bearbeitet wird. Namentlich kann es bei Radien von weniger als 0.1 mm aufgrund der Kerbwirkung während dem Sintern zu Rissen im Implantat kommen.

Das Keramikmaterial, aus dem die Gelenkschale gefertigt ist, kann osteokonduktive Eigenschaften aufweisen. Der Begriff osteokonduktiv bezieht sich in diesem Zusammenhang darauf, dass ein osteokonduktives Material eine Stützstruktur für die Knochenzellen bilden kann, wodurch das natürliche Knochenwachstum begünstigt wird. Entsprechend unterstützt ein osteokonduktives Schalenmaterial aus Keramik den Heilungsprozess des Knochens nach einer Implantation, sowie die Bildung einer stabilen Verbindung zwischen Schalenimplantat und dem Knochen.

Das Keramikmaterial, aus dem die Gelenkschale gefertigt ist, kann aus einer osteokonduktiven Oxid-Sinterkeramik, vorzugsweise aus einer Zirkoniumoxid-Keramik oder einer Aluminiumoxid-Zirkoniumoxid-Mischkeramik bestehen. Derartige Materialien zeichnen sich durch eine hervorragende Biokompatibilität, eine gute Bearbeitbarkeit und eine hohe mechanische Festigkeit aus.

Bevorzugt weist die konvexe Aussenfläche der Gelenkschale eine osteokonduktive Beschichtung auf. Dadurch wird es ermöglicht, das keramische Schalenmaterial rein nach Kriterien der mechanischen Festigkeit auszuwählen und eine optimale Osteokonduktivität durch die Auswahl eines geeigneten Beschichtungsmaterials zu erzielen.

Die osteokonduktive Beschichtung kann eine Mikrostrukturierung aufweisen. Eine derartige Mikrostrukturierung kann beispielsweise eine Porenstruktur mit einer Vielzahl von Hohlräumen umfassen, die eine Grösse von 0.003 mm bis zu 0.3 mm haben.
Die osteokonduktive Beschichtung kann, unter anderem durch die erwähnte Mikrostrukturierung, zusätzlich osteointegrativ sein. Der Begriff einer osteointegrativen Oberfläche bezieht sich in diesem Zusammenhang auf eine Oberfläche, die einen direkten funktionellen und strukturellen Verbund mit dem organisierten, lebenden Knochengewebe eingehen kann. Dadurch kann eine äusserst feste Verankerung des Implantates in einem dafür vorgesehenen Knochen erzielt werden.

Die osteokonduktive, vorzugsweise auch osteointegrative, Beschichtung kann aus einem osteokonduktiven Metall, vorzugsweise aus einem osteokonduktiven Übergangsmetall bestehen. Metallische Beschichtungen sind besonders gut dazu geeignet, auf keramische Oberflächen aufgetragen zu werden. Als mögliche Beschichtungsverfahren kommen dafür Plasmabeschichtung, Abscheiden aus der Dampfphase (Chemical Vapor Deposition, Physical Vapor Deposition), Sputtering, Sol-Gel-Abscheidung, Ausfällung oder Elektrophorese in Frage. So kann die osteokonduktive Beschichtung aus Platin oder aus einem Element der zweiten oder dritten Nebengruppe, vorzugsweise aus Titan, Zirkonium, Niob oder Tantal bestehen. Derartige Materialien sind für Implantate im klinischen Umfeld weit verbreitet.

Ferner kann die osteokonduktive, vorzugsweise auch osteointegrative, Beschichtung aus Hydroxylapatit oder aus einer osteokonduktiven Oxid-Sinterkeramik, vorzugsweise aus einer Zirkoniumoxid-Keramik oder einer Aluminiumoxid-Zirkoniumoxid-Mischkeramik bestehen. Keramische Beschichtungen sind für das besagte Implantat besonders gut geeignet, da sie bei der Herstellung desselben auf den Grünling aufgetragen werden können, wodurch sie während des Sinterprozesses einen Stoffschluss mit ihm eingehen. Zudem zeichnen sich diese Materialien durch eine hervorragende Biokompatibilität und sehr gute osteokonduktive bzw. osteointegrative Eigenschaften aus.

Das Keramikmaterial, aus dem die Gelenkschale gefertigt ist, kann aus einer Oxid-Sinterkeramik, vorzugsweise einer Aluminiumoxid-Keramik, einer Zirkoniumoxid-Keramik oder einer Aluminiumoxid-Zirkoniumoxid-Mischkeramik bestehen. Diese Materialien lassen sich im Zuge des Herstellungsverfahrens kostengünstig und effizient bearbeiten, insbesondere beschichten. Ferner ermöglichen sie es, eine Gelenkschale in hoher Präzision zu fertigen, die eine überaus hohe mechanische Belastbarkeit aufweist.

Des Weiteren ist erfindungsgemäss das Keramikmaterial, aus dem die Gelenkschale gefertigt ist, ausgewählt aus einer Liste bestehend aus Titancarbid-Keramik, Titanoxycarbid-Keramik, Titannitrid-Keramik, Titancarbonitrid-Keramik, Titanaluminiumnitrid-Keramik, Titanaluminiumcarbonitrid-Keramik, Titanzirkoniumnitrid-Keramik und Siliziumnitrid-Keramik. Diese Materialien zeichnen sich durch eine überaus grosse Härte aus, was der Gelenkschale eine sehr hohe Steifigkeit gibt und darüber hinaus bei einer einteiligen Gelenkschale das Gleitverhalten einer Gelenkkugel auf einer konkaven Lagerfläche verbessern kann.

Weitere Vorteile und Einzelmerkmale der Erfindung ergeben sich aus der nachfolgenden Beschreibung mehrerer Ausführungsbeispiele und aus den Figuren.

Es zeigen schematisch:
- Figur 1:: Aufsicht auf eine erfindungsgemässe Gelenkschale;
- Figur 2:: Schnitt durch eine Gelenkschale gemäss Figur 1;
- Figur 3:: Schnitt durch ein alternatives Ausführungsbeispiel einer erfindungsgemässen Gelenkschale;
- Figur 4:: Aufsicht auf eine erfindungsgemässe Gelenkschale mit spiralförmigen Rillen;
- Figur 5:: Seitenansicht einer Gelenkschale gemäss Figur 4;
- Figur 6:: eine perspektivische Ansicht einer weiteren Ausführungsform einer erfindungsgemässen Gelenkschale;
- Figur 7:: Detailansicht des Oberflächenprofils einer Gelenkschale gemäss Figur 6;
- Figur 8:: Aufsicht auf eine weitere Ausführungsform einer erfindungsgemässen Gelenkschale mit gegenläufigen spiralförmigen Rillen;
- Figur 9:: Seitenansicht einer Gelenkschale gemäss Figur 8;
- Figur 10:: Schnittbild durch eine erfindungsgemässe Gelenkschale mit Beschichtung;
- Figur 11:: Schnittbild durch eine alternative Ausführungsform einer erfindungsgemässen Gelenkschale mit Beschichtung;
- Figur 12:: Teilvergrösserung eines Schnittbildes durch eine Gelenkschale gemäss Figur 11.

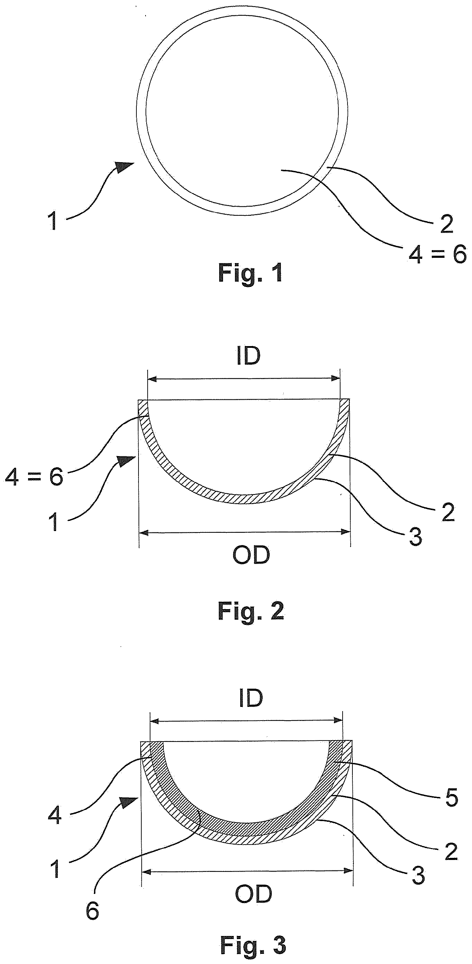

Figur 1 zeigt eine Aufsicht auf eine einteilige Gelenkpfanne 1 mit einer erfindungsgemässen Gelenkschale. Die konkave Lagerfläche 4 bildet hier gleichzeitig auch die Lagerfläche 6 für eine in die Gelenkpfanne 1 einzusetzende Gelenkkugel.

Figur 2 zeigt einen Schnitt durch eine einteilige Gelenkpfanne 1 mit einer erfindungsgemässen Gelenkschale 2 gemäss Figur 1. Diese ist in Form einer Kugelkalottenschale mit einer konvexen Aussenfläche 3 und einer konkaven Lagerfläche 4 ausgestaltet. Die Gelenkschale hat einen äusseren Durchmesser (OD) und einen inneren Durchmesser (ID).

Figur 3 zeigt einen Schnitt durch eine mehrteilige Gelenkpfanne 1 mit einer erfindungsgemässen Gelenkschale 2, in welche eine Innenschale 5 eingesetzt ist. Diese Innenschale 5 ist fest mit der konkaven Innenfläche 4 der Gelenkschale 2 verbunden und bildet eine Lagerfläche 11 für eine in die Gelenkpfanne 1 einzusetzende Gelenkkugel.

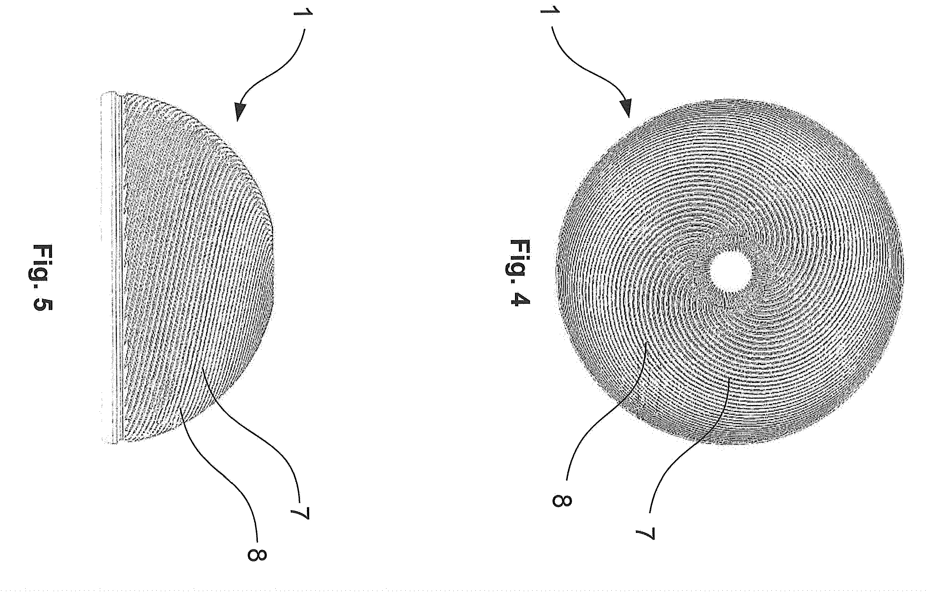

Figuren 4 und 5 zeigen eine Aufsicht und eine Seitenansicht auf ein Ausführungsbeispiel einer erfindungsgemässen Gelenkschale 2. Dabei umfasst die Makrostrukturierung auf der konvexen Aussenseite der Schale eine Vielzahl von Strukturelementen 7, die durch Vertiefungen 8, in diesem Fall durch spiralförmige Rillen, begrenzt sind.

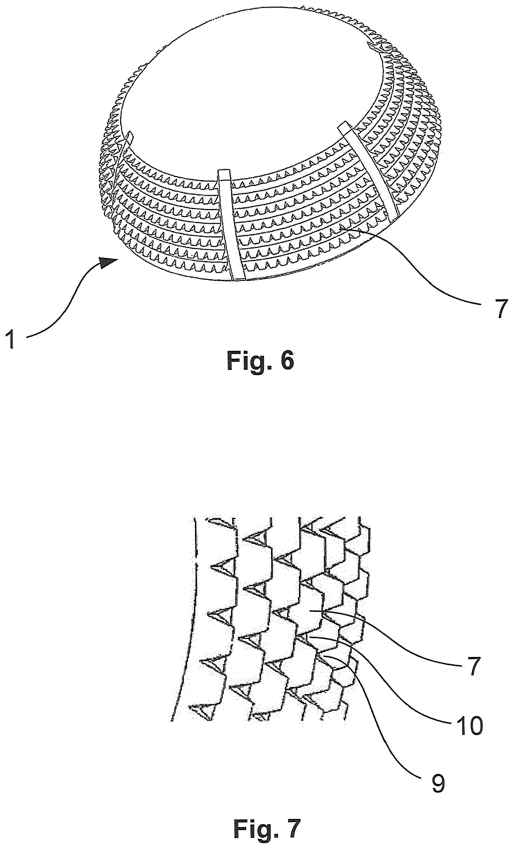

Figur 6 zeigt eine perspektivische Darstellung einer alternativen Ausführungsform einer erfindungsgemässen Gelenkpfanne 2. In Figur 7 ist das Oberflächenprofil der besagten Gelenkpfanne 2 vergrössert dargestellt. Es ist zu erkennen, dass die Vertiefungen 9, welche Strukturelemente 7 in einer Reihe voneinander trennen, gegenüber Vertiefungen 10, welche Strukturelemente 7 in einer benachbarten Reihe voneinander trennen, relativ zu einander versetzt angeordnet sind.

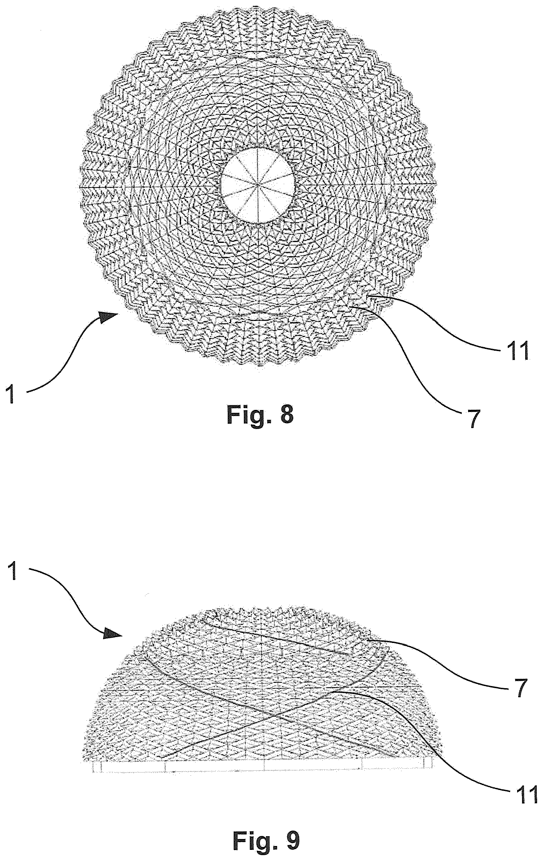

Die Figuren 8 und 9 zeigen eine Aufsicht bzw. eine Seitenansicht auf eine weitere alternative Ausführungsform einer erfindungsgemässen Gelenkpfanne 2. Die Vertiefungen 11, welche die Strukturelemente 7 begrenzen, sind hier durch sich überkreuzende Rillen mit entgegengesetzter Steigung gebildet.

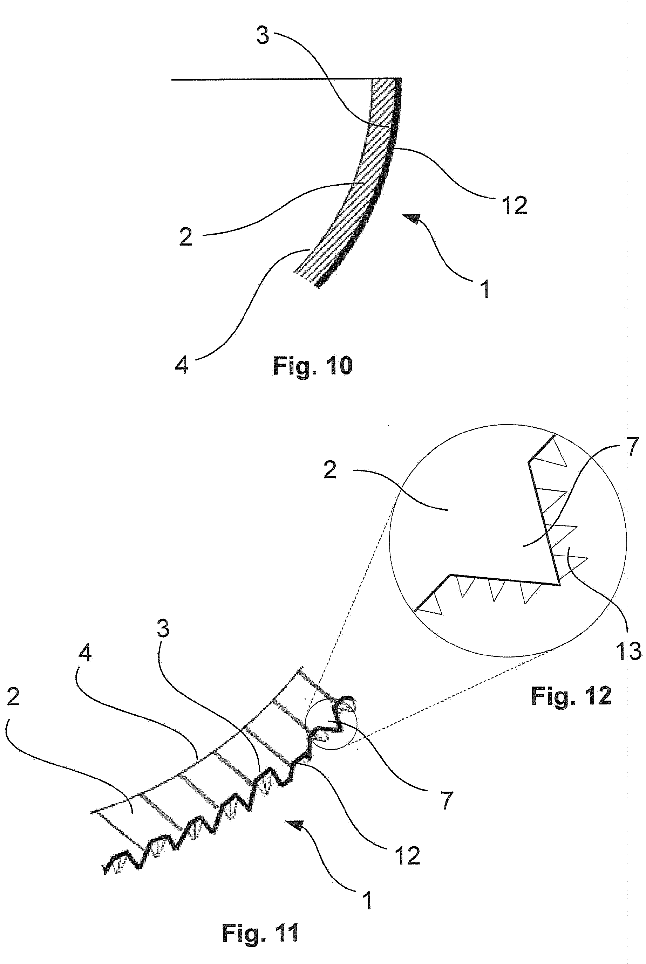

Figur 10 zeigt einen Teil eines Schnittes durch eine erfindungsgemässe Gelenkpfanne 2 mit glatter konvexer Aussenseite 3. Die dargestellte Gelenkschale 2 besitzt die Form einer Kugelkalottenschale. Die konvexe Aussenfläche 3 weist dabei eine osteokonduktive Beschichtung 12 auf. Die konkave Lagerfläche 4 ist hingegen unbeschichtet.

Figur 11 zeigt einen Teil eines Schnittes eines weiteren alternativen Ausführungsbeispiels einer erfindungsgemässen Gelenkschale 2. In diesem Fall weist die Gelenkschale 2 eine makrostrukturierte Aussenfläche 3 auf. Diese Aussenfläche 3 ist mit einer osteokonduktiven Beschichtung 12 versehen. Die konkave Innenfläche 4 auf der Innenseite der Kugelkalottenschale ist hingegen auch hier unbeschichtet.

Figur 12 zeigt eine Teilvergrösserung eines Schnittes durch eine erfindungsgemässen Gelenkschale 2 gemäss Figur 11. Es ist zu erkennen, dass sich auf den Strukturelementen 7 der Makrostrukturierung eine osteokonduktive Beschichtung 12 befindet, welche wiederum Poren 13 aufweist. Durch diese Poren kann die Oberfläche einen direkten funktionellen und strukturellen Verbund mit dem Knochengewebe eingehen.

## Patentansprüche

1. Künstliche Gelenkschale (2), insbesondere Hüftgelenkschale, zur Implantation in eine Kavität in einem Knochen, insbesondere im Wesentlichen in Form einer Kugelkalottenschale, mit einer konvexen Aussenfläche (3) und einer konkaven Innenfläche (4), mit einem äusseren Durchmesser (OD) und einem inneren Durchmesser (ID), wobei die Gelenkschale (2) aus einem Keramikmaterial gefertigt ist und die konvexe Aussenfläche (3) eine Makrostrukturierung aufweist, **dadurch gekennzeichnet, dass** das Verhältnis der Differenz (D) zwischen äusserem Durchmesser (OD) und innerem Durchmesser (ID) zum äusseren Durchmesser (OD) in einem Bereich zwischen 0.2 und 0.1 liegt und das Keramikmaterial, aus dem die Gelenkschale (2) gefertigt ist, ausgewählt ist aus der Liste bestehend aus Titancarbid-Keramik, Titanoxycarbid-Keramik, Titannitrid-Keramik, Titancarbonitrid-Keramik, Titanaluminiumnitrid-Keramik, Titanaluminiumcarbonitrid-Keramik und Titanzirkoniumnitrid-Keramik.

2. Gelenkschale (2) nach Anspruch 1, **dadurch gekennzeichnet, dass** die konkave Innenfläche (4) eine Lagerfläche (6) für eine in der Gelenkschale (1) aufzunehmende Gelenkkugel bildet.

3. Gelenkschale (2) nach Anspruch 1, **dadurch gekennzeichnet, dass** die konkave Innenfläche (4) eine Haltefläche für eine in der Gelenkschale (2) aufzunehmende Innenschale (5) bildet.

4. Gelenkschale (2) nach einem der Ansprüche 1 bis 3, **dadurch gekennzeichnet, dass** die Makrostrukturierung eine Vielzahl von Strukturelementen (7) umfasst, die durch Vertiefungen (8), vorzugsweise durch Materialabtragungen, begrenzt sind und eine Grösse von 0.3 mm bis zu 3 mm haben.

5. Gelenkschale (2) nach einem der Ansprüche 1 bis 3, **dadurch gekennzeichnet, dass** die Makrostrukturierung eine Porenstruktur mit einer Vielzahl von Hohlräumen umfasst, die eine Grösse von 0.03 mm bis zu 3 mm haben.

6. Gelenkschale (2) nach einem der Ansprüche 1 bis 5, **dadurch gekennzeichnet, dass** die Makrostrukturierung zum Einwachsen der Trabekelstruktur eines Knochens geeignet ist.

7. Gelenkschale (2) nach Anspruch 4, **dadurch gekennzeichnet, dass** Vertiefungen (9), insbesondere Materialabtragungen, welche Strukturelemente (7) in einer Reihe voneinander trennen, gegenüber Vertiefungen (10), insbesondere Materialabtragungen, welche Strukturelemente in einer benachbarten Reihe voneinander trennen, relativ zueinander versetzt angeordnet sind.

8. Gelenkschale (2) nach Anspruch 4, **dadurch gekennzeichnet, dass** Vertiefungen (11), insbesondere Materialabtragungen, welche die Strukturelemente begrenzen, durch sich überkreuzende Rillen mit entgegengesetzter Steigung gebildet sind.

9. Gelenkschale (2) nach einem der Ansprüche 4, 7 oder 8, **dadurch gekennzeichnet, dass** die Strukturelemente (7) als Quader, Pyramiden, Tetraeder, Keile, Polyeder oder der Form eines spitzwinkligen Satteldachs geformt sind.

10. Gelenkschale (2) nach Anspruch 4 oder einem der Ansprüche 7 bis 9, **dadurch gekennzeichnet, dass** durch die Vertiefungen (8, 9, 10, 11), insbesondere Materialabtragungen, konkave Oberflächenbereiche gebildet werden, deren Krümmung einen Krümmungskreisradius von mindestens 0.1 mm hat.

11. Gelenkschale (2) nach einem der Ansprüche 1 bis 10, **dadurch gekennzeichnet, dass** das Keramikmaterial, aus dem die Gelenkschale (2) gefertigt ist, osteokonduktive Eigenschaften aufweist.

12. Gelenkschale (2) nach Anspruch 11, **dadurch gekennzeichnet, dass** das Keramikmaterial, aus dem die Gelenkschale (2) gefertigt ist, aus einer osteokonduktiven Oxid-Sinterkeramik, vorzugsweise aus einer Zirkoniumoxid-Keramik oder einer Aluminiumoxid-Zirkoniumoxid-Mischkeramik, besteht.

13. Gelenkschale (2) nach einem der Ansprüche 1 bis 10, **dadurch gekennzeichnet, dass** die konvexe Aussenfläche (3) eine osteokonduktive Beschichtung (12) aufweist.

14. Gelenkschale (2) nach Anspruch 13, **dadurch gekennzeichnet, dass** die osteokonduktive Beschichtung (12) eine Mikrostrukturierung aufweist.

15. Gelenkschale (2) nach Anspruch 14, **dadurch gekennzeichnet, dass** die Mikrostrukturierung eine Porenstruktur mit einer Vielzahl von Hohlräumen (13) umfasst, die eine Grösse von 0.003 mm bis zu 0.3 mm haben.

16. Gelenkschale (2) nach einem der Ansprüche 13 bis 15, **dadurch gekennzeichnet, dass** die osteokonduktive Beschichtung (12) zusätzlich osteointegrativ ist.

17. Gelenkschale (2) nach einem der Ansprüche 13 bis 16, **dadurch gekennzeichnet, dass** die osteokonduktive, vorzugsweise auch osteointegrative, Beschichtung (12) aus einem osteokonduktiven Metall, vorzugsweise aus einem osteokonduktiven Übergangsmetall besteht.

18. Gelenkschale (2) nach Anspruch 17, **dadurch gekennzeichnet, dass** die osteokonduktive, vorzugsweise auch osteointegrative, Beschichtung (12) aus Platin oder aus einem Element der zweiten oder dritten Nebengruppe, vorzugsweise aus Titan, Zirkonium, Niob oder Tantal, besteht.

19. Gelenkschale (2) nach einem der Ansprüche 13 bis 16, **dadurch gekennzeichnet, dass** die osteokonduktive, vorzugsweise auch osteointegrative, Beschichtung (12) aus Hydroxylapatit oder aus einer osteokonduktiven Oxid-Sinterkeramik, vorzugsweise aus einer Zirkoniumoxid-Keramik oder einer Aluminiumoxid-Zirkoniumoxid-Mischkeramik, besteht.

20. Gelenkschale (2) nach einem der Ansprüche 13 bis 19, **dadurch gekennzeichnet, dass** das Keramikmaterial, aus dem die Gelenkschale (2) gefertigt ist, aus einer Oxid-Sinterkeramik, vorzugsweise aus einer Aluminiumoxid-Keramik, einer Zirkoniumoxid-Keramik oder einer Aluminiumoxid-Zirkoniumoxid-Mischkeramik, besteht.

## Claims

1. Artificial joint cup (2), in particular hip joint cup, for implanting in a cavity in a bone, in particular substantially in the form of a spherical dome cup, having a convex outer surface (3) and a concave inner surface (4), having an outer diameter (OD) and an inner diameter (ID), wherein the joint cup (2) is made of a ceramic material, and the convex outer surface (3) has a macro-structuring **characterized in that** the ratio of the difference (D) between outer diameter (OD) and inner diameter (ID) in relation to the outer diameter (OD) lies in a range between 0.2 and 0.1 and the ceramic material from which the joint cup (2) is made is chosen from the list consisting of titanium carbide ceramic, titanium oxycarbide ceramic, titanium nitride ceramic, titanium carbonitride ceramic, titanium aluminum nitride ceramic, titanium aluminum carbonitride ceramic and titanium zirconium nitride ceramic.

2. Joint cup (2) according to Claim 1, **characterized in that** the concave inner surface (4) forms a bearing surface (6) for a joint ball that is to be received in the joint cup (2).

3. Joint cup (2) according to Claim 1, **characterized in that** the concave inner surface (4) forms a holding surface for an inner cup (5) that is to be received in the joint cup (2).

4. Joint cup (2) according to one of Claims 1 to 3, **characterized in that** the macro-structuring comprises a multiplicity of structure elements (7) which are delimited by depressions (8), preferably by material ablations, and measure from 0.3 mm to 3 mm.

5. Joint cup (2) according to one of Claims 1 to 3, **characterized in that** the macro-structuring comprises a pore structure with a multiplicity of cavities that measure from 0.03 mm to 3 mm.

6. Joint cup (2) according to one of Claims 1 to 5, **characterized in that** the macro-structuring is suitable for the inward growth of the trabecular structure of a bone.

7. Joint cup (2) according to Claim 4, **characterized in that** depressions (9), in particular material ablations, which separate structure elements (7) in one row from each other, are arranged offset relative to each other in relation to depressions (10), in particular material ablations, which separate structure elements in an adjacent row from each other.

8. Joint cup (2) according to Claim 4, **characterized in that** depressions (11), in particular material ablations, which delimit the structure elements are formed by intersecting grooves of mutually opposite pitch.

9. Joint cup (2) according to one of Claims 4, 7 and 8, **characterized in that** the structure elements (7) are shaped as cubes, pyramids, tetrahedrons, wedges, polyhedrons or have the shape of an acuteangled pitched roof.

10. Joint cup (2) according to Claim 4 or one of Claims 7 to 9, **characterized in that** the depressions (8, 9, 10, 11), in particular material ablations, form concave surface areas, of which the curvature has a radius of curvature of at least 0.1 mm.

11. Joint cup (2) according to one of Claims 1 to 10, **characterized in that** the ceramic material from which the joint cup (2) is made has osteoconductive properties.

12. Joint cup (2) according to Claim 11, **characterized in that** the ceramic material from which the joint cup (2) is made consists of an osteoconductive sintered oxide ceramic, preferably a zirconium oxide ceramic or an aluminum oxide/zirconium oxide mixed ceramic.

13. Joint cup (2) according to one of Claims 1 to 10, **characterized in that** the convex outer surface (3) has an osteoconductive coating (12).

14. Joint cup (2) according to Claim 13, **characterized in that** the osteoconductive coating (12) has a micro-structuring.

15. Joint cup (2) according to Claim 14, **characterized in that** the micro-structuring comprises a pore structure with a multiplicity of cavities (13) that measure from 0.003 mm to 0.3 mm.

16. Joint cup (2) according to one of Claims 13 to 15, **characterized in that** the osteoconductive coating (12) is additionally osteointegrative.

17. Joint cup (2) according to one of Claims 13 to 16, **characterized in that** the osteoconductive and preferably also osteointegrative coating (12) consists of an osteoconductive metal, preferably an osteoconductive transition metal.

18. Joint cup (2) according to Claim 17, **characterized in that** the osteoconductive and preferably also osteointegrative coating (12) consists of platinum or of an element of the second or third transition group, preferably titanium, zirconium, niobium or tantalum.

19. Joint cup (2) according to one of Claims 13 to 16, **characterized in that** the osteoconductive and preferably also osteointegrative coating (12) consists of hydroxyapatite or of an osteoconductive sintered oxide ceramic, preferably a zirconium oxide ceramic or an aluminum oxide/zirconium oxide mixed ceramic.

20. Joint cup (2) according to one of Claims 13 to 19, **characterized in that** the ceramic material from which the joint cup (2) is made consists of a sintered oxide ceramic, preferably an aluminum oxide ceramic, a zirconium oxide ceramic, or an aluminum oxide/zirconium oxide mixed ceramic.

## Revendications

1. Cupule articulaire (2) artificielle, en particulier cupule acétabulaire, pour une implantation dans une cavité dans un os, en particulier essentiellement sous forme d'une cupule à calotte sphérique, présentant une surface externe (3) convexe et une surface interne (4) concave, présentant un diamètre externe (OD) et un diamètre interne (ID), la cupule articulaire (2) étant fabriquée en un matériau céramique et la surface externe convexe (3) présentant une macrostructuration, **caractérisée en ce que** le rapport de la différence (D) entre le diamètre externe (OD) et le diamètre interne (ID) au diamètre externe (OD) se situe dans une plage entre 0.2 et 0.1 et le matériau céramique, à partir duquel la cupule articulaire (2) est fabriquée, est choisi dans la liste constituée par la céramique de carbure de titane, la céramique d'oxycarbure de titane, la céramique de nitrure de titane, la céramique de carbonitrure de titane, la céramique de nitrure de titane et d'aluminium, la céramique de carbonitrure de titane et d'aluminium et la céramique de nitrure de titane et de zirconium.

2. Cupule articulaire (2) selon la revendication 1, **caractérisée en ce que** la surface interne concave (4) forme une surface d'appui (6) pour une bille articulaire à loger dans la cupule articulaire (2).

3. Cupule articulaire (2) selon la revendication 1, **caractérisée en ce que** la surface interne concave (4) forme une surface de fixation pour une cupule interne (5) à loger dans la cupule articulaire (2).

4. Cupule articulaire (2) selon l'une quelconque des revendications 1 à 3, **caractérisée en ce que** la macrostructuration comprend une multitude d'éléments structuraux (7), qui sont délimités par des creux (8), de préférence par des enlèvements de matière, et qui présentent une dimension de 0.3 mm jusqu'à 3 mm.

5. Cupule articulaire (2) selon l'une quelconque des revendications 1 à 3, **caractérisée en ce que** la macrostructuration comprend une structure de pores présentant une multitude d'espaces creux, qui présentent une dimension de 0.03 mm jusqu'à 3 mm.

6. Cupule articulaire (2) selon l'une quelconque des revendications 1 à 5, **caractérisée en ce que** la macrostructuration est appropriée pour une croissance avec intégration dans la structure trabéculaire d'un os.

7. Cupule articulaire (2) selon la revendication 4, **caractérisée en ce que** des creux (9), en particulier des enlèvements de matière, qui séparent les uns des autres des éléments structuraux (7) dans une ligne, par rapport à des creux (10), en particulier des enlèvements de matière, qui séparent les uns des autres des éléments structuraux dans une ligne adjacente, sont disposés de manière décalée les uns par rapport aux autres.

8. Cupule articulaire (2) selon la revendication 4, **caractérisée en ce que** des creux (11), en particulier des enlèvements de matière, qui délimitent les éléments structuraux, sont formés par des rainures qui se croisent avec une pente opposée.

9. Cupule articulaire (2) selon l'une quelconque des revendications 4, 7 ou 8, **caractérisée en ce que** les éléments structuraux (7) sont formés sous forme de parallélépipèdes rectangles, de pyramides, de tétraèdres, de coins, de polyèdres ou sous forme d'un toit en pente à angle aigu.

10. Cupule articulaire (2) selon la revendication 4 ou selon l'une quelconque des revendications 7 à 9, **caractérisée en ce que** les creux (8, 9, 10, 11), en particulier des enlèvements de matière, forment des zones de surface concaves, dont la courbure présente un rayon de courbure d'au moins 0.1 mm.

11. Cupule articulaire (2) selon l'une quelconque des revendications 1 à 10, **caractérisée en ce que** le matériau céramique, à partir duquel la cupule articulaire (2) est fabriquée, présente des propriétés ostéoconductrices.

12. Cupule articulaire (2) selon la revendication 11, **caractérisée en ce que** le matériau céramique, à partir duquel la cupule articulaire (2) est fabriquée, est constitué par une céramique d'oxyde frittée ostéoconductrice, de préférence par une céramique d'oxyde de zirconium ou une céramique mixte d'oxyde d'aluminium-oxyde de zirconium.

13. Cupule articulaire (2) selon l'une quelconque des revendications 1 à 10, **caractérisée en ce que** la surface externe convexe (3) présente un revêtement (12) ostéoconducteur.

14. Cupule articulaire (2) selon la revendication 13, **caractérisée en ce que** le revêtement ostéoconducteur (12) présente une microstructuration.

15. Cupule articulaire (2) selon la revendication 14, **caractérisée en ce que** la microstructuration comprend une structure de pores présentant une multitude d'espaces creux (13), qui présentent une dimension de 0.003 mm jusqu'à 0.3 mm.

16. Cupule articulaire (2) selon l'une quelconque des revendications 13 à 15, **caractérisée en ce que** le revêtement ostéoconducteur (12) est en outre ostéo-intégrant.

17. Cupule articulaire (2) selon l'une quelconque des revendications 13 à 16, **caractérisée en ce que** le revêtement (12) ostéoconducteur, de préférence également ostéo-intégrant, est constitué par un métal ostéoconducteur, en particulier par un métal de transition ostéoconducteur.

18. Cupule articulaire (2) selon la revendication 17, **caractérisée en ce que** le revêtement (12) ostéoconducteur, de préférence également ostéo-intégrant, est constitué par du platine ou par un élément du deuxième ou du troisième groupe secondaire, de préférence par du titane, du zirconium, du niobium ou du tantale.

19. Cupule articulaire (2) selon l'une quelconque des revendications 13 à 16, **caractérisée en ce que** le revêtement (12) ostéoconducteur, de préférence également ostéo-intégrant, est constitué par de l'hydroxyapatite ou par une céramique d'oxyde frittée ostéoconductrice, de préférence par une céramique d'oxyde de zirconium ou une céramique mixte d'oxyde d'aluminium-oxyde de zirconium.

20. Cupule articulaire (2) selon l'une quelconque des revendications 13 à 19, **caractérisée en ce que** le matériau céramique, à partir duquel la cupule articulaire (2) est fabriquée, est constitué par une céramique d'oxyde frittée, de préférence par une céramique d'oxyde d'aluminium, une céramique d'oxyde de zirconium ou une céramique mixte d'oxyde d'aluminium-oxyde de zirconium.
